# EUROPEAN PATENT SPECIFICATION

(11) **EP 3 052 077 B1**
(45) Date of publication and mention of the grant of the patent: **19.09.2018**
(21) Application number: 14850602.5
(22) Date of filing: 30.09.2014
(51) Int. Cl.: A61K 8/97, A61K 8/99, A61K 8/30, A61Q 19/00

(54) **TOPICAL PREPARATIONS COMPRISING GRAPE SEED EXTRACT**
TOPISCHE PRÄPARATE MIT TRAUBENKERNEXTRAKT
PRÉPARATIONS TOPIQUES COMPRENANT DES EXTRAITS DE PÉPINS DE RAISIN

(30) Priority: 03.10.2013 US 201314045487
(43) Date of publication of application: 10.08.2016
(73) Proprietor: ELC Management LLC, Melville, NY 11747 (US)
(72) Inventor: LEE, Wilson A., Hauppauge, New York 11788 (US); CROCKETT, Barbara A., Greenlawn, New York 11740 (US); PELLE, Edward, Valley Stream, New York 11580 (US); PERNODET, Nadine A., Huntington Station, New York 11746 (US); KEUPP, Georgena M., Bay Shore, New York 11706 (US)
(74) Representative: Hirsch & Associés
(86) International application number: PCT/US2014/058360
(87) International publication number: WO 2015/050883

(56) References cited:
- WO-A1-2013/055315
- CN-A- 102 755 281
- US-A1- 2007 202 215
- US-A1- 2009 110 674
- US-A1- 2010 291 050
- US-A1- 2011 206 721
- "Vitis vinifera (Grape) Ingredients as Used in Cosmetics", , 21 February 2012 (2012-02-21), XP002767756, Retrieved from the Internet: URL:http://www.cir-safety.org/sites/defaul t/files/vitis022012SLR_for%20posting.pdf [retrieved on 2017-03-02]
- R. Debowska et al.: "Foilc acid (folacin) - New Application of a Cosmetic ingredient", Kosmetische Medizin, vol. 3 2 August 2005 (2005-08-02), pages 16-22, XP002767757, Retrieved from the Internet: URL:http://www.eris.pl/biuletyn_images/art 2005d.pdf [retrieved on 2017-03-02]
- Christoph Huschka: "Untersuchungen zur Wirkung von Biotin auf humane Keratinozyten und zur Modulation der Biotinpenetration in humane Haut", , 8 December 2000 (2000-12-08), XP002767758, Retrieved from the Internet: URL:https://sundoc.bibliothek.uni-halle.de /diss-online/98/99H014/prom.pdf [retrieved on 2017-03-02]
- ANITHA, T.: 'Medicinal plants used in skin protection' ASIAN JOURNAL OF PHARMACEUTICAL AND CLINICAL RESEARCH vol. 5, no. SUPPL., 2012, pages 40 - 43, XP055336765

## Description

### Field of the Invention

The invention is in the field of skin care, specifically directed to topical products comprising grape seed and at least some of folic acid, biotin, *Bifidobacterium longum* extract and *Echinacea purpurea.*

### Background

The effects of environmental insult to human skin are well documented. Exposure to sunlight and various polluting factors in the environment causes the generation of reactive oxygen intermediates that contribute to acute and chronic aging of the skin. As a result, topical preparations that contain antioxidants that scavenge reactive oxygen species (ROS) in the skin have become common. In general, the topical application of products that contain antioxidants remains an important area of research in the skin care field.

Of course, there are a great many materials that provide various sorts of activity and efficacy to skin care products. These are often combined to provide multiple types of benefits in a single product. However, being active chemical agents, it is not uncommon for these materials to interfere with each other, negating some or all of the intended efficacy. Thus, it is no small challenge to formulate safe, stable and efficacious products at commercially viable costs.

The antioxidant activity of grape seed (Vitis vinifera) extract in topical formulations is known. Grape seed extracts are also used as emollients, emulsifiers and a skin conditioning agents (see for example "Vitis vinifera (Grape) Ingredients as Used in Cosmetics", retrievable from the Internet under URL http://www.cir-safety.org/sites/ default/files/vitis022012SLR_for%20posting.pdf).

Folic acid (CAS 59-30-3) is an organic compound with formula (C₁₉H₁₉N₇O₆): 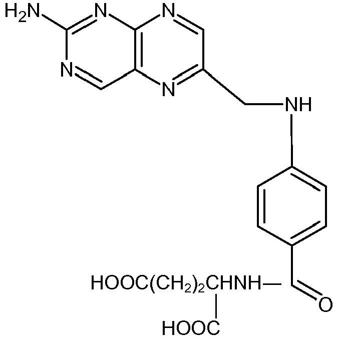 Folic acid is used in topical preparations as a skin conditioning agent.

Biotin (CAS 58-85-5) is known to be used as a hair and skin conditioning agent in topical formulations, such shampoo, conditioner, soap, bath oils and salts, and makeup. Biotin is a carboxylic acid of the formula (C₁₀H₁₆N₂O₃S):

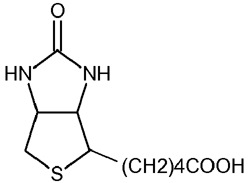

Bifidus or Bifida ferment extract is an extract obtained from the fermentation of *Bifidobacterium longum.* It has been used as a skin conditioning agent and humectant. It is also reported to promote the DNA repair process of skin cells. For example, US4,464,362 discloses the topical use of species *Bifidobacterium longum* (Reuter) to treat photodamage.

*Echinacea purpurea* (Coneflower) extract is known to be used in topical compositions, including hair products, where it is reported to possess anti-bacterial and anti-inflammatory activity. *Echinacea purpurea,* should not be confused with *Echinacea angustifolia, Echinacea pallid* and other varieties of *Echinacea.*

We have noted that commercially viable compositions comprising grape seed extract and one or more of folic acid, biotin, bifidus, and *Echinacea purpurea* have widely varying antioxidant activities. Each of these ingredients offers useful benefits in the battle against environmentally induced skin damage. We suspected that in a base formulation, these five ingredients were interfering in complex ways, sometimes giving a topical composition with useful antioxidant activity, sometimes not. The problem therefore, was to discover one or more optimal blends of these five ingredients in a safe, stable topical skin care preparation.

### Detailed Description of the Invention

The present invention thus relates to topical compositions comprising grape seed extract, as described in the claims.

The antioxidation activity of various topical preparations was measured with the following assay.

### Assay Preparation

For a complete description of the technique, see E. Pelle et al., "A test for antioxidant activity in cosmetic formulations," in Journal of Cosmetic Science 53:237-240 (2002). The Randox Assay for Total Antioxidant Status Kit (Randox; Antrim, United Kingdom) is used for this assay. 2, 2'-azino-di-(3-ethylbenzthiazoline sulphonate) (ATBS) is reacted with a peroxidase and H₂O₂ to convert ATBS into a radical cation. In this state, ATBS forms a chromogen that can be measured spectrophotometrically at 600 nm. However, in the presence of antioxidants, this color formation is inhibited. Thus, the absence of or a reduction in the 600 nm signal is indicative of antioxidant activity.

### Test Sample Preparation

Each topical composition to be tested is diluted to 1% in isopropyl alcohol. Typically, 50-100µl of this dilution is further diluted with water up to 250µl.

### Assay

1.5 ml of the chromogen solution is added to a test sample, followed by the addition of 0.3 ml of substrate solution. The absorbance (A) of the test samples is immediately measured in a Beckman DU-7500 spectrophotometer using a kinetics/time program.

### Calculations

Percent inhibition of oxidation is calculated as (dA_{control} - dA_{product}/dA_{control}) x 100 and used to quantitate IC₅₀ values, where dA is the change in absorbance over two minutes. 15 to 85 nanomoles of an antioxidant standard (AO std; 6-hydroxy-2,5,7,8-tetramethylchroman-2-carboxylic acid) are used to determine the relative activity of a test sample. In the following tables, the column labeled "Control" shows a base formula for a topical hair care preparation. The other columns (labeled 1-8 and 10-11) show the base formula combined with various combinations of the actives: grape seed extract, bifidus extract, folic acid, *Echinacea purpurea* and biotin. We report the percent inhibition of oxidation.

### Results

Tables 1a and 1b show eleven formulae that were tested for anti-oxidant activity according to the assay described above.

The base formula, labeled "Control", offers 0% antioxidant activity. Formula 8, which is the base formula plus 0.05% grape seed extract (a known antioxidant), offers about 17.5% inhibition of oxidation.

At this point, we wished to include in the formulation, bifidus extract, folic acid, *Echinacea purpurea* and biotin, for their known benefits. When we did this (formula 1) something unexpected happened. Unexpectedly, the antioxidant activity was significantly reduced (to about 5% inhibition of oxidation). Various combinations of the active ingredients were tested to determine which formulations gave significant antioxidant activity.

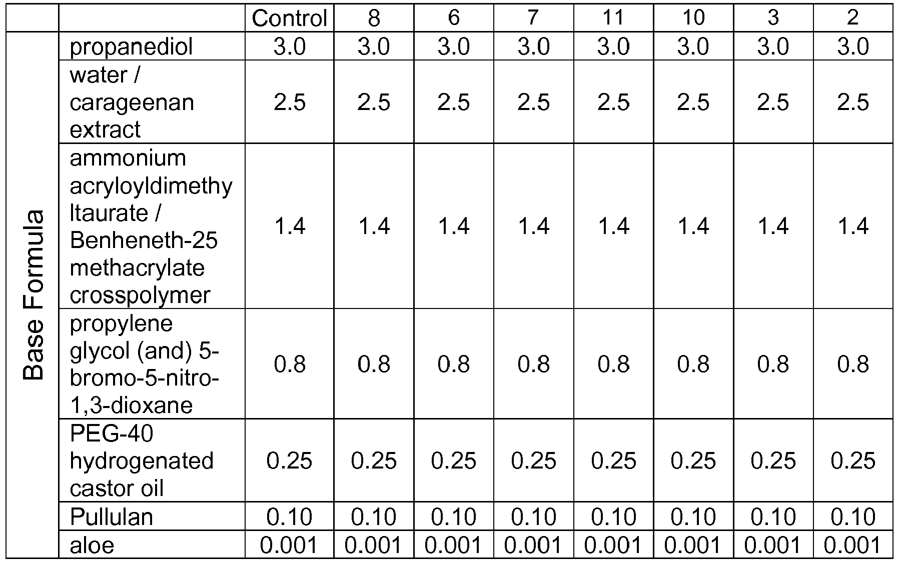

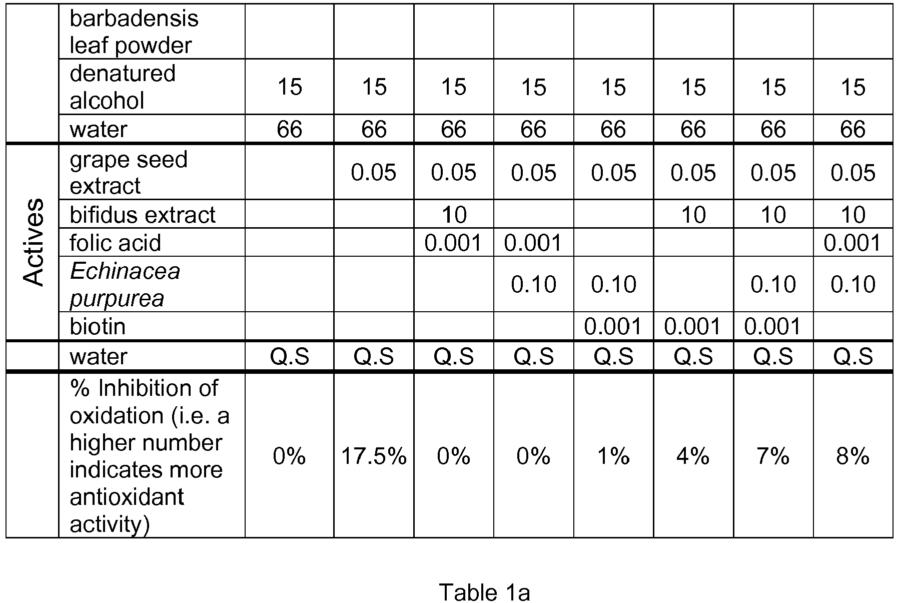

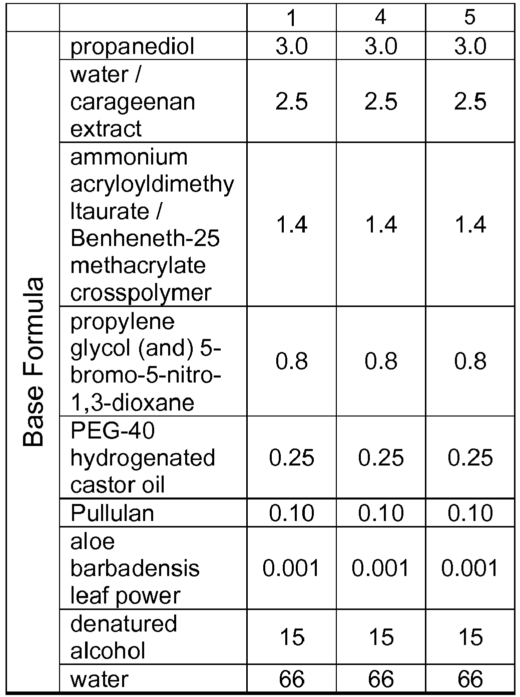

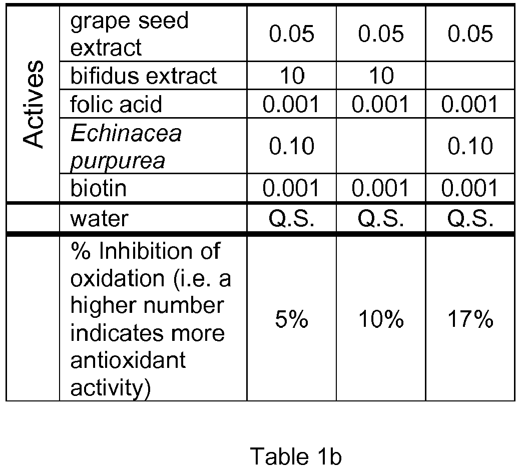

### Grape Seed Extract Plus Combinations of Three

Comparing formula 3 to formula 1, folic acid seems to reduce the antioxidant activity of the grape seed extract.

Comparing formula 2 to formula 1, biotin seems to reduce the antioxidant activity of the grape seed extract.

Comparing formula 4 to formula 1, *Echinacea purpurea* seems to reduce the antioxidant activity of the grape seed extract.

Comparing formula 5 to formula 1, bifidus seems to reduce the antioxidant activity of the grape seed extract.

It seems that each one of bifidus extract, folic acid, *Echinacea purpurea* and biotin, reduces the antioxidant activity of grape seed extract, when the other three are also present. Put another way, as regards antioxidant activity of grape seed extract, it is better to have a formulation with any three of bifidus extract, folic acid, *Echinacea purpurea* and biotin, rather than all four. This is a wholly unexpected result, and supports a claim such as, "A topical composition comprising grape seed extract and exactly three of bifidus extract, folic acid, *Echinacea purpurea* and biotin."

### Grape Seed Extract Plus Combinations of Two

### Folic acid and Bifidus

Comparing formula 6 to formula 8, the combination of folic acid and bifidus significantly reduces (in this case eliminates) the antioxidant activity of grape seed extract. However, comparing formula 11 to formula 1, the combination of folic acid and bifidus seems to boost the antioxidant activity of the grape seed extract when biotin and *Echinacea purpurea* are also present.

### Folic acid and Echinacea purpurea

Comparing formula 7 to formula 8, the combination of folic acid and *Echinacea purpurea* significantly reduces (in this case eliminates) the antioxidant activity of grape seed extract. However, comparing formula 10 to formula 1 the combination of folic acid and *Echinacea purpurea* seems to have little effect (maybe a slight boost) on antioxidant activity of the grape seed extract when biotin and bifidus are also present.

### Biotin and Echinacea purpurea

Comparing formula 11 to formula 8, the combination of biotin and *Echinacea purpurea* significantly reduces the antioxidant activity of grape seed extract. However, comparing formula 6 to formula 1, the combination of biotin and *Echinacea purpurea* seems to boost the antioxidant activity of the grape seed extract when folic acid and bifidus are also present.

### Biotin and Bifidus

Comparing formula 10 to formula 8, the combination of biotin and bifidus significantly reduces the antioxidant activity of grape seed extract. However, comparing formula 7 to formula 1, the combination of biotin and bifidus seems to boost the antioxidant activity of the grape seed extract when folic acid and *Echinacea purpurea* are also present.

So, the following combinations: folic acid /bifidus, biotin/*Echinacea purpurea,* biotin/ bifidus, and folic acid/*Echinacea purpurea*, significantly reduce the antioxidant activity of grape seed extract, unless the other two actives are also present, in which case there is a boost in antioxidant activity of the grape seed extract, but not as much as when exactly three of the actives are present. Thus, the data supports the following claims.

A topical composition comprising grape seed extract and a combination of folic acid and bifidus, but not the combination of biotin and *Echinacea purpurea.*

A topical composition comprising grape seed extract and a combination of folic acid and bifidus, but not the combination of biotin and *Echinacea purpurea.*

A topical composition comprising grape seed extract and a combination of biotin and *Echinacea purpurea,* but not the combination of folic acid and bifidus.

A topical composition comprising grape seed extract and a combination of biotin and bifidus, but not the combination of folic acid/*Echinacea purpurea.*

A topical composition comprising grape seed extract and a combination of folic acid and *Echinacea purpurea,* but not the combination of biotin and bifidus.

### Discussion

What is interesting about these results is that the antioxidant activities of the different grape seed formulations point to different synergistic behaviors between combinations of the same four actives. Therefore, in formulating an anti-oxidant skin care composition that incorporates the known benefits of grape seed extract, bifidus extract, folic acid, *Echinacea purpurea* and biotin, care must be taken to blend the actives in combinations that do not destroy all of the antioxidant activity.

We have determined that, as regards compositions comprising grape seed extract, the combination of exactly three of the following: folic acid, biotin, bifidus and *Echinacea purpurea* is useful; the combination of biotin, bifidus and *Echinacea purpurea* (without folic acid) is preferred, as is the combination of folic acid, bifidus and *Echinacea purpurea* (without biotin); the combination of folic acid, biotin and bifidus (without *Echinacea purpurea*) is more preferred; and the combination of folic acid, biotin and *Echinacea purpurea* (without bifidus, formula 5, for example) is most preferred.

### Relative Concentrations

In compositions of the present invention, the relative concentrations of certain ingredients are selected according to the following:
The ratio of grape seed to folic acid may vary from 50:0 to 50:1.
The ratio of grape seed to biotin may vary from 50:0 to 50:1.
The ratio of grape seed to *Echinacea purpurea* may vary from 50:0 to 50:100.
The ratio of grape seed to bifidus extract may vary from 50:0 to 50:10,000.

When all five ingredients are present in a composition which is not according to the present invention, then the most preferred ratio of concentrations of folic acid to biotin to grape seed to *Echinacea purpurea* to bifidus extract is 1 : 1 : 50 : 100 : 10,000, with no more than 10% variation in any of the ingredients. This may be written as: 0.9 - 1.1 : 0.9 - 1.1 : 45 - 55 : 90 - 110 : 9,000 - 11,000 (folic acid : biotin : grape seed : *Echinacea purpurea* : bifidus extract). So, one exemplary ratio would be: 0.001 gm of folic acid, 0.001 gm of biotin, 0.05 gm of grape seed extract, 0.10 gm of *Echinacea purpurea* and 10.0 gm of bifidus extract.

Compositions according to the present invention may be implemented as skin care preparations, hair care preparations, or as color cosmetics. Color cosmetics include blush, eyeshadow, lipstick, lip gloss, foundation, concealer, and the like, for altering the appearance of the skin. Compositions according to the present invention may be implemented as lotions, creams toners, gels, solid or semi-solid preparations. Skin care preparations include those that target the face, hands, and scalp. Hair care preparations include those that target the hair of the head or the eyelashes. Compositions may be in the form of emulsions, suspensions or mixtures.

The compositions may contain virtually any cosmetically acceptable ingredients. Many hair and /or skin conditioning agents may be mentioned, but for scalp products the following are especially useful: adenosine phosphate, aloe barbadensis, Arabidopsis thaliana extract, arginine, butylene glycol, caffeine, caprylyl glycol, carrageenan, ethylhexylglycerine, soybean extract, hydrolyzed wheat protein, lactobacillus ferment, laminaria saccharina extract, panthenine, pullulan, quinoa seed extract, rosemary leaf extract, saccharomyces lysate extract, sodium RNA fragments, yeast extract. These may typically be used at concentrations 0.0001 to 5.0% by weight of the final composition. It is preferred to use at least three of these for an effective scalp treatment. At least five of these is more preferred for addressing the needs of the scalp.

Useful solvents include water and/or alcohol. Total solvents may typically comprise 60% - 90% of the composition by weight. Alcohol may typically range from 0% - 20% by weight of the composition. Useful preservatives include benzoic acid, chlorphenesin, phenoxyethanol, potassium sorbate, sodium benzoate, sodium dehydroacetate and sorbic acid. Total preservatives may range from 0.01% to 5.0% by weight of the composition. The composition may comprise: emulsion facilitators such as ammonium acryloyldimethyltaurate / Benheneth-25 methacrylate crosspolymer and lecithin; pH adjusters, such as ammonium stearate; humectants such as panthenol and saccharide isomerate; binders, such as hydrogenated castor oil; bulking agents, such as silica; absorbents, such as maltodextrin; antioxidants (other than grape seed extract), such as tocopherol.

The following are non-limiting examples of scalp conditioning products not according to the present invention. Ingredients are given as percent by weight of the final composition.

| Ingredient | Example 1 | Example 2 | Example 3 | Example 4 |
|---|---|---|---|---|
| alcohol | -- | -- | 15.000 | -- |
| water/chondrus crispus extract | 3.000 | 3.000 | 2.500 | 3.000 |
| pullulan | 0.100 | 0.100 | 0.100 | 0.100 |
| aloe vera powder | 0.010 | 0.100 | 0.001 | 0.001 |
| hydrolyzed wheat protein | 0.010 | 0.010 | 0.500 | 0.500 |
| hydrolyzed soy protein | -- | -- | 0.500 | 0.500 |
| quinoa protein | 0.010 | 0.010 | -- | -- |
| **folic acid** | 0.001 | 0.001 | 0.001 | 0.001 |
| **biotin** | 0.001 | 0.001 | 0.001 | 0.001 |
| **vitis vinifera (grape) seed extract** | 0.050 | 0.050 | 0.050 | 0.100 |
| panthenol | 0.100 | 0.100 | 0.200 | 0.200 |
| sodium dehydroacetate | 0.200 | 0.200 | -- | -- |
| silica | 0.060 | 0.050 | -- | -- |
| saccharide isomerate | 0.500 | 0.500 | -- | -- |
| **bifida ferment lysate** | 10.000 | 10.000 | 10.000 | 10.000 |
| glycine soya (soybean) extract / bifida ferment lysate | 0.500 | 0.500 | 0.500 | 0.500 |
| ***Echinacea purpurea* (coneflower) extract** | 0.100 | 0.100 | 0.100 | 0.100 |
| arginine | 0.010 | 0.100 | 0.100 | 0.100 |
| caffeine | 0.050 | 0.200 | 0.050 | 0.050 |
| sodium ribonucleic acid | 0.010 | 0.100 | 0.010 | 0.010 |
| water / Arabidopsis thaliana extract | 0.100 | 0.100 | 0.100 | 0.100 |
| lactobacillus ferment / lecithin / water | 0.050 | 0.050 | 0.050 | 0.050 |
| PEG-40 hydrogenated castor oil | 0.200 | 0.200 | 0.250 | 0.250 |
| caprylyl glycol | 0.100 | 0.100 | -- | -- |
| ethylhexylglycerine | 0.100 | 0.100 | -- | -- |
| phenoxyethanol | 0.800 | 0.800 | 0.800 | 0.800 |
| propanediol | -- | -- | 3.000 | 2.000 |
| ammonium acryloyldimethyltaurate / beheneth-25 methacrylate crosspolymer | 0.800 | 0.800 | 1.400 | 1.300 |
| water/butylene glycol / laminara saccharina extract | -- | 0.500 | -- | -- |
| yeast (saccharomyces cerevisiae) | -- | 0.300 | -- | -- |
| adenosine phosphate | -- | 0.040 | -- | -- |
| pantethine | -- | 0.005 | -- | -- |
| bisabolol | -- | -- | 0.100 | 0.100 |
| magnolia extract | -- | -- | 0.010 | 0.010 |
| gluconolactone/sodium benzoate/ calcium gluconate | | | | 0.700 |
| water | Q.S. | Q.S. | Q.S. | Q.S. |

Examples 1 - 4 (not according to the present invention) have grape seed plus the four actives, folic acid, biotin, *bifidus* and *Echinacea purpurea.* Examples according to the present invention may be created by removing any one of folic acid, biotin, *bifidus* or *Echinacea purpurea* from examples 1 - 4, while retaining the other three. Additional examples may also be created by varying the relative concentrations of grape seed, folic acid, biotin, *bifidus* and *Echinacea purpurea* within the guidelines for relative concentration presented above.

## Claims

1. A topical composition comprising grape seed extract and exactly three of the following: folic acid, biotin, bifidus ferment extract and Echinacea purpurea.

2. The composition of claim 1 comprising folic acid, biotin, and bifidus ferment extract.

3. The composition of claim 2 wherein the following relative concentrations are adhered to:
the ratio of grape seed to folic acid may vary from 50:0 to 50:1;
the ratio of grape seed to biotin may vary from 50:0 to 50:1; and
the ratio of grape seed to bifidus extract may vary from 50:0 to 50:10,000.

4. The composition of claim 1 comprising folic acid, biotin, and Echinacea purpurea.

5. The composition of claim 4 wherein the following relative concentrations are adhered to:
the ratio of grape seed to folic acid may vary from 50:0 to 50:1;
the ratio of grape seed to biotin may vary from 50:0 to 50:1; and
the ratio of grape seed to *Echinacea purpurea* may vary from 50:0 to 50:100.

6. The composition of claim 1 comprising folic acid, bifidus ferment extract and Echinacea purpurea.

7. The composition of claim 6 wherein the following relative concentrations are adhered to:
the ratio of grape seed to folic acid may vary from 50:0 to 50:1;
the ratio of grape seed to *Echinacea purpurea* may vary from 50:0 to 50:100;
and the ratio of grape seed to bifidus extract may vary from 50:0 to 50:10,000.

8. The composition of claim 1 comprising biotin, bifidus ferment extract and *Echinacea purpurea.*

9. The composition of claim 8 wherein the following relative concentrations are adhered to:
the ratio of grape seed to biotin may vary from 50:0 to 50:1;
the ratio of grape seed to *Echinacea purpurea* may vary from 50:0 to 50:100;
and the ratio of grape seed to bifidus extract may vary from 50:0 to 50:10,000.

10. The topical composition of claim 1 further comprising:
at least three of the following conditioning agents at concentrations of 0.001 to 5.0% by weight of the final composition: adenosine phosphate, aloe barbadensis, Arabidopsis thaliana extract, arginine, butylene glycol, caffeine, caprylyl glycol, carrageenan, ethylhexylglycerine, soybean extract, hydrolyzed wheat protein, lactobacillus ferment, laminaria saccharina extract, panthenine, pullulan, quinoa seed extract, rosemary leaf extract, saccharomyces lysate extract, sodium RNA fragments, yeast extract.

11. The topical composition of claim 10 comprising at least five of the conditioning agents.

## Patentansprüche

1. Topische Zusammensetzung umfassend Traubenkernextrakt und genau drei der Folgenden: Folsäure, Biotin, Bifidus-Fermentextrakt und Purpur-Sonnenhut.

2. Zusammensetzung nach Anspruch 1, umfassend Folsäure, Biotin und Bifidus-Fermentextrakt.

3. Zusammensetzung nach Anspruch 2, wobei die folgenden relativen Konzentrationen eingehalten werden:
das Verhältnis von Traubenkern zu Folsäure kann von 50:0 bis 50:1 variieren;
das Verhältnis von Traubenkern zu Biotin kann von 50:0 bis 50:1 variieren; und
das Verhältnis von Traubenkern zu Bifidus-Extrakt kann von 50:0 bis 50:10.000 variieren.

4. Zusammensetzung nach Anspruch 1 umfassend Folsäure, Biotin und Purpur-Sonnenhut.

5. Zusammensetzung nach Anspruch 4, wobei die folgenden relativen Konzentrationen eingehalten werden:
das Verhältnis von Traubenkern zu Folsäure kann von 50:0 bis 50:1 variieren;
das Verhältnis von Traubenkern zu Biotin kann von 50:0 bis 50:1 variieren; und
das Verhältnis von Traubenkern zu Purpur-Sonnenhut kann von 50:0 bis 50:100 variieren.

6. Zusammensetzung nach Anspruch 1 umfassend Folsäure, Bifidus-Fermentextrakt und Purpur-Sonnenhut.

7. Zusammensetzung nach Anspruch 6, wobei die folgenden relativen Konzentrationen eingehalten werden:
das Verhältnis von Traubenkern zu Folsäure kann von 50:0 bis 50:1 variieren;
das Verhältnis von Traubenkern zu Purpur-Sonnenhut kann von 50:0 bis 50:100 variieren; und
das Verhältnis von Traubenkern zu Bifidus-Extrakt kann von 50:0 bis 50:10.000 variieren.

8. Zusammensetzung nach Anspruch 1, umfassend Biotin, Bifidus-Fermentextrakt und Purpur-Sonnenhut.

9. Zusammensetzung nach Anspruch 8, wobei die folgenden relativen Konzentrationen eingehalten werden:
das Verhältnis von Traubenkern zu Biotin kann von 50:0 bis 50:1 variieren;
das Verhältnis von Traubenkern zu Purpur-Sonnenhut kann von 50:0 bis 50:100 variieren; und
das Verhältnis von Traubenkern zu Bifidus-Extrakt kann von 50:0 bis 50:10.000 variieren.

10. Topische Zusammensetzung nach Anspruch 1, ferner Folgendes umfassend:
mindestens drei der folgenden Konditioniermittel in Konzentrationen von 0,001 bis 5,0 Gew.-% der endgültigen Zusammensetzung: Adenosinphosphat, Aloe barbadensis, Arabidopsis thaliana-Extrakt, Arginin, Butylenglycol, Coffein, Caprylylglycol, Carrageen, Ethylhexylglycerin, Sojabohnenextrakt, hydrolysiertes Weizenprotein, Lactobacillus-Ferment, Laminaria saccharina-Extrakt, Panthenin, Pullulan, Quinoakörnerextrakt, Rosmarinblätterextrakt, Saccharomyceslysat-Extrakt, Natrium-RNA-Fragmente, Hefeextrakt.

11. Topische Zusammensetzung nach Anspruch 10, umfassend mindestens fünf der Konditioniermittel.

## Revendications

1. Composition topique comprenant un extrait de pépins de raisin et exactement trois des suivants : l'acide folique, la biotine, l'extrait de ferment de bifidus et *Echinacea purpurea.*

2. Composition selon la revendication 1 comprenant de l'acide folique, de la biotine, et de l'extrait de ferment de bifidus.

3. Composition selon la revendication 2 dans laquelle les concentrations relatives suivantes sont respectées :
le rapport des pépins de raisin sur l'acide folique peut varier de 50/0 à 50/1 ;
le rapport des pépins de raisin sur la biotine peut varier de 50/0 à 50/1 ; et
le rapport des pépins de raisin sur l'extrait de bifidus peut varier de 50/0 à 50/10 000.

4. Composition selon la revendication 1 comprenant de l'acide folique, de la biotine, et *Echinacea purpurea.*

5. Composition selon la revendication 4 dans laquelle les concentrations relatives suivantes sont respectées :
le rapport des pépins de raisin sur l'acide folique peut varier de 50/0 à 50/1 ;
le rapport des pépins de raisin sur la biotine peut varier de 50/0 à 50/1 ; et
le rapport des pépins de raisin sur *Echinacea purpurea* peut varier de 50/0 à 50/100.

6. Composition selon la revendication 1 comprenant de l'acide folique, de l'extrait de ferment de bifidus et *Echinacea purpurea.*

7. Composition selon la revendication 6 dans laquelle les concentrations relatives suivantes sont respectées :
le rapport des pépins de raisin sur l'acide folique peut varier de 50/0 à 50/1 ;
le rapport des pépins de raisin sur *Echinacea purpurea* peut varier de 50/0 à 50/100 ; et
le rapport des pépins de raisin sur l'extrait de bifidus peut varier de 50/0 à 50/10 000.

8. Composition selon la revendication 1 comprenant de la biotine, de l'extrait de ferment de bifidus et *Echinacea purpurea.*

9. Composition selon la revendication 8 dans laquelle les concentrations relatives suivantes sont respectées :
le rapport des pépins de raisin sur la biotine peut varier de 50/0 à 50/1 ;
le rapport des pépins de raisin sur *Echinacea purpurea* peut varier de 50/0 à 50/100 ; et
le rapport des pépins de raisin sur l'extrait de bifidus peut varier de 50/0 à 50/10 000.

10. Composition topique selon la revendication 1 comprenant en outre :
au moins trois des agents de conditionnement suivants à des concentrations de 0,001 à 5,0 % en poids de la composition finale : adénosine phosphate, *Aloe barbadensis*, extrait d'*Arabidopsis thaliana*, arginine, butylène glycol, caféine, caprylyl-glycol, carraghénane, éthylhexyl-glycérine, extrait de soja, protéine de blé hydrolysée, ferment de *Lactobacillus*, extrait de *Laminaria saccharina*, panthénine, pullulane, extrait de graines de quinoa, extrait de feuilles de romarin, extrait de lysat de *Saccharomyces*, fragments d'ARN au sodium, extrait de levure.

11. Composition topique selon la revendication 10 comprenant au moins cinq des agents de conditionnement.
